(19) Europäisches Patentamt
European Patent Office
Office européen des brevets

(11) Publication number: **0 420 450 B1**

(12) **EUROPEAN PATENT SPECIFICATION**

(45) Date of publication of patent specification: **06.12.95**  (51) Int. Cl.⁶: **C12M 1/30**

(21) Application number: **90309967.9**

(22) Date of filing: **12.09.90**

(54) **Collection and transportation device for microorganisms.**

(30) Priority: **18.09.89 NO 893706**

(43) Date of publication of application:
**03.04.91 Bulletin 91/14**

(45) Publication of the grant of the patent:
**06.12.95 Bulletin 95/49**

(84) Designated Contracting States:
**AT BE CH DE DK ES FR GB GR IT LI LU NL SE**

(56) References cited:
FR-A- 2 612 297    GB-A- 945 190
US-A- 3 913 564    US-A- 3 939 044
US-A- 4 140 489    US-A- 4 150 950

(73) Proprietor: **BIONOR A/S**
**Stromdaljordet 4**
**P.O. Box 1868 Gulset**
**N-3701 Skien (NO)**

(72) Inventor: **Hagen, Norman**
**Gulsetkasa 5,**
**3700 Skien (NO)**
Inventor: **Kristiansen, Bjorn-Erik**
**Stromdalasen 60,**
**3700 Skien (NO)**
Inventor: **Sorensen, Birger**
**Tuyrveueb 37,**
**3700 Skien (NO)**
Inventor: **Kotlar, Hans Kristian**
**Hubroveien 8A,**
**3700 Skien (NO)**

(74) Representative: **Allen, Oliver John Richard et al**
**Lloyd Wise, Tregear & Co.**
**Norman House**
**105-109 Strand**
**London, WC2R 0AE (GB)**

## Description

This invention relates to devices for the collection and transportation of microorganisms, for example for samples taken by or for medical doctors, veterinaries and laboratory personnel.

After a sample is taken, it is important that the microorganisms are kept alive and not permitted contact with any kind of contamination, extraneous matters and the like before the sample is taken for analysis and identification.

Samples of microorganisms are traditionally transported in a glass tube and sealed by a screw cork which is filled with a standard, sterile transport medium. To protect the glass tube against breakage and leakage, it is placed within a two piece plastic cover. To obtain the sample itself special sample swabs are utilised. This is a bulky and elaborate operation.

Therefore, other and more simple solutions have been proposed. One of these is described in US patent no. 3,450,129. The transport means of the invention of that patent consists of a unit in the form of a flexible plastics tube of polypropylene or the like with a removable lid which contains a separate glass ampulla filled with a sterile transport medium. The complete unit is covered by a protective seal made of plastic material.

When a sample is to be taken, the air-tight seal is broken and the lid with the swab is removed to collect a bacterium specimen. The lid with the swab is placed back in the tube and subsequently the glass ampulla is broken so that the swab is moistened by the culture medium. To ensure complete moistening the swab is in the form of an absorbent plug.

This device has gained wide utilisation in a number of countries but has a number of drawbacks. The glass ampulla can be broken accidentally, the sterile conditions are difficult to maintain after the original seal is broken and the device is complex and costly.

US-A-3939044 discloses an anaerobic microbe transport assembly comprising two nested tubes having an elastomeric sealing plug to the centre of which a swab is attached.

GB-A-945150 discloses a container for storing sterile specimens comprising a tube closed by a cap which is provided with means to support a swab stick.

US-A-4150950 discloses a system for transporting clinical specimentts having a tube which has a screw cap to which a swab is attached, the action of screwing the cap onto the tube causing the end of the swab to rupture a seal so as to immerse the end of the swab in a liquid preservative.

US-A-4140489 discloses a test tube having an axial inner tube of a dark colour or into which a dark background can be introduced to enable microrganisms contained between the test tube and the inner tube to be counted easily.

FR-A-2612297 discloses a laboratory container comprising two compartments and adapted to allow two constituents, one of which is solid and the other liquid, to come into contact.

Another proposal for a safer collection and transport device is described in US patent no. 3.890,204. This construction consists of a sealed glass tube containing the culture medium and a separate lid carrying a sample swab. The two separate devices are packed together in a sealed package. The glass tube is air and gas-proof and closed at both ends.

One of the ends is provided with indentations so that the end piece can be broken and the sample placed in the culture medium. When the sample has been taken the swab is placed inside the tube and the handle, which is also used as a lid, fits tightly around the tube to close it.

The glass tube is then placed back into the original packaging and forwarded to the laboratory for analysis of the sample. With such a device the need for using an absorbent plug and an extra outer plastic tube as in US 3,450,129, is eliminated. However, this solution is not satisfactory either. The glass tube can easily break during transport and the cover or lid may not seal the tube satisfactorily.

Other solutions have been proposed to eliminate the drawbacks of using breakable glass ampullae. Thus it has been proposed to use soft plastics, such as polyolefins and polyurethanes. Such materials are of course unbreakable, but the softness creates problems under transport and handling. The culture medium may be displaced or even disappear from the tube. Furthermore, the plastic materials are not diffusion resistant so that a culture medium will be contaminated after a short period of exposure. So far, therefore, the use of glass tubes and glass ampullae has prevailed.

A collection and transportation device for cultures of microorganisms according to the invention comprises a vessel of 'unbreakable', transparent plastics material containing a part of a sample swab of the type comprising a breakable handle and a swab of absorbent fibres for gathering a micro-organism culture and closing means for the vessel which includes means for releasably retaining the swab characterized in that the vessel comprises an open-ended tube (1) of diffusion-resistant material having a coefficient of permeability for $O_2$ < 300 and with a mechanical impact strength such that no cracks are formed in the material when subjected to an impact force of 60 Kj at 20°C the closing means (2) also being made of an 'unbreakable' and diffusion-resistant material and in that the length of the tube (1) is less than the length of the unbroken sample swab, the swab having being broken to a length approximately that of the tube (1), and culture medium being provided within the tube (1).

The main tube is made of an impact-resistant, diffusion-resistant and transparent material, preferably polyamide or polyethylene terephtalate which are nearly diffusion-resistant for gases like oxygen, nitrogen and carbon dioxide. Other suitable plastic materials which are also nearly diffusion-resistant are polyvinylidenechloride and polyvinylfluoride or other materials which satisfy a permeability for $O_2$ better or comparable to the above mentioned plastics. For instance, polyamide and polyethylene terephtalate are nearly 30 times as resistant to diffusion, at room temperature, than polyolefins. Plastics like polystyrene and polypropylene are on the other hand not applicable, having a coefficient of permeability which is 10-100 times higher than the aforementioned plastics. The plastic employed should have a permeability for oxygen at 25° which is lower than that which is limited by a permeability coefficient of :

$$P \times \underline{\frac{cm^3(NTP) \times 100\mu m}{m^2 \times 24h \times bar}} = 300,$$

but preferably the coefficient should be below 30, to guarantee the complete durability and validity of the reduced medium.

The plastics used should, in addition, have a mechanical shock resistance better or comparable to the demands by DIN 53 453, which means that no breaks or marks are obtained when the material is subjected to an impact of 60KJ at 20°C.

Such a microorganism collection and transport device has the advantages of easy availability and safe keeping of the culture medium under satisfactorily sterile conditions. It provides a simple and cheap construction which is unbreakable under normal circumstances and gives a safe and reliable protection without the danger of leakage of possibly pathogenic organisms after the samples have been collected.

The device is biologically safe, sterile and diffusion resistant under all conditions before, during and after the sample-taking procedure.

The device of the invention provides a tube which is permanently closed and in which the bacterium sample is completely covered by the culture medium when it is introduced into this medium by means of a sample swab. The system ensures the survival of both aerobic and anaerobic microorganisms.

The open end of the tube may be provided with external screw threads and the closing means with complementary internal screw threads. The closing means may also include means for retaining the upper end of the shaft of a swab, so that as the closing means is screwed onto the tube, the swab rotates within the culture medium, to provide complete covering of the swab with the culture medium.

Additional means may be provided around the open end of the tube to ensure a diffusion-tight seal. This means may comprise a surface which extends around the circumference of the tube, adjacent its open end, which co-operates with the bottom edge of the closing means. An O-ring may be provided between the abutting surfaces to improve further the seal.

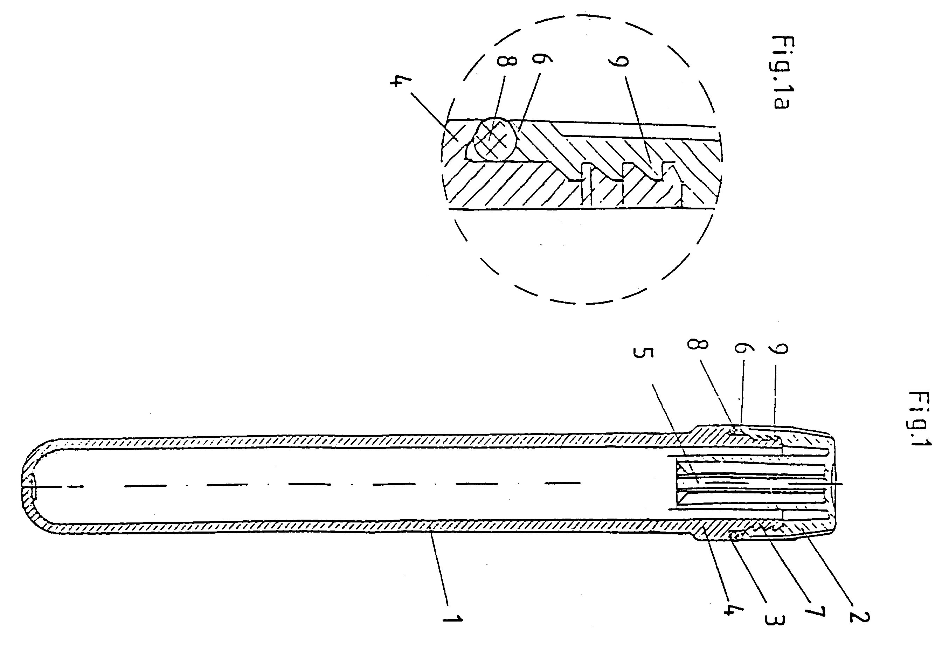

The invention will now be further described with reference to the accompanying drawings in which: Figure 1 is a cross section of an example of a collection and transport device according to the invention; figure 1a is a detailed view of one end of the device shown in figure 1;

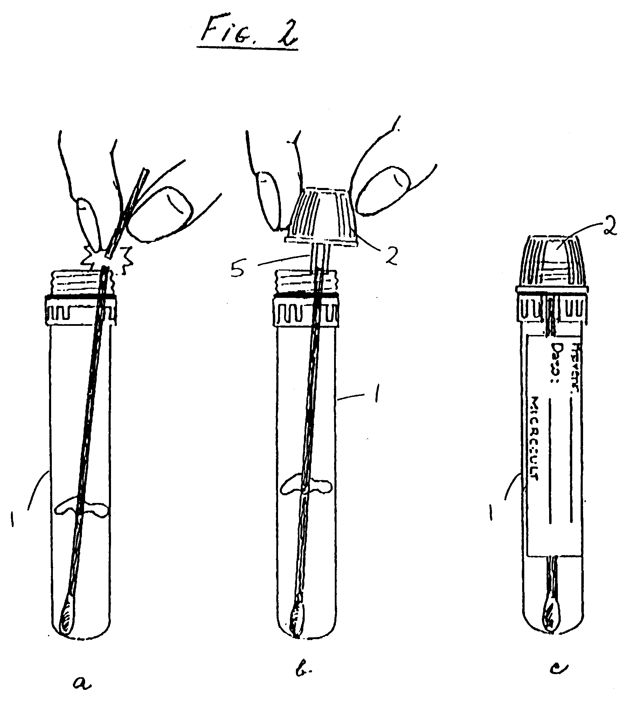

Figures 2a, b and c are schematic views of the device to illustrate how it is to be used.

The device comprises a tube 1 and a lid 2. The open end of the tube 1 is provided with screw threads 7 and a collar 4 having an upper contact surface 3 for carrying a seal in the form of an O-ring 8 or the like. A fitting lid 2 is provided with internal threads 9 and a central hollow end-piece 5 which can accept and retain the handle of a swab (see fig. 2b). The edge 6 of the lid is bevelled, giving it a sharp outer edge which will be pressed tightly into the O-ring 8 to form a diffusion-resistant seal when the lid is screwed on tightly. Complete diffusion sealage will be obtained when the lid is fastened manually (see fig.1a).

**Examples for use of the transport device**

The tube is filled with a suitable culture medium such as for example STUARTS medium.

The sample swab, as shown in fig. 2a, comprises a breakable plastic handle or the like and a swab of absorbent cotton fibres. Each swab is provided in a separate breakable package and is sterile.

The sterile package is intended to be broken just before the sample is to be taken. During the sample-taking the tube will remain closed and sterile. When the sample has been collected, the screw lid of the tube is opened, the swab is inserted into the tube 1, the handle part of the sample swab is broken against the edge of the tube (as shown in fig. 2a) and the end is inserted into the end-piece 5 of the screw lid (as shown in fig.2b), after which the lid is screwed on tightly (see fig.2c).

This results in a rotating movement of the swab within the culture medium which will result in a complete covering of the swab so that when it is retracted to perform the final analysis, air or other contaminants would not have been able to enter.

In addition to the culture medium, the tube can be filled with an inert gas such as nitrogen.

The short time interval during which the tube will be open to the atmosphere, prevents the device from being contaminated by extraneous agents. When the lid or cork is tightened the tube is completely sealed. The tube is not susceptible to breakage and transportation can take place without special precautions.

The device is considerably improved as compared with prior known construction.

It is more hygienic, safer to transport due to the use of shock-resistant plastic, more durable due to its diffusion resistance, less bulky and suitable for use without outer protective packaging

The collection and transport device can be provided with a number of different culture media, both selective and universal. Also media which promote selective growth during transport can be employed. Examples are :
  i) GC-media
  ii) Listeria media
  iii) ASTM-media for fungi
  iv) CBI for Clostridium fungi
  v) CLED for Coli bacteria
  vi) THIO-media for anaerobic fungus
  vii) etc.

## Claims

1. A collection and transportation device for cultures of microorganisms comprising a vessel of 'unbreakable', transparent plastics material containing a part of a sample swab of the type comprising a breakable handle and a swab of absorbent fibres for gathering a microorganism culture, and closing means for the vessel which includes means for releasably retaining the swab characterized in that the vessel comprises an open-ended tube (1) of diffusion-resistant material having a coefficient of permeability for $0_2 < 300$ and with a mechanical impact strength such that no cracks are formed in the material when subjected to an impact force of 60 kj at 20°C the closing means (2) also being made of an 'unbreakable' and diffusion-resistant material and in that the length of the tube (1) is less than the length of the unbroken sample swab, the swab having being broken to a length approximately that of the tube (1), and culture medium being provided within the tube (1).

2. A collection and transport device according to Claim 1 characterized in that the plastics material is polyamide or polyethylene terephtalate.

3. A collection and transport device according to any preceding claim characterized in that the open end of the tube (1) is provided with an external screw thread and the corresponding closing means with an internal screw thread and in that the closing means is provided with a central hollow retaining piece (5) which is designed to carry the upper end of the shaft of a swab whereby the swab is rotated into the culture medium when the closing means is tightened onto the tube (1).

4. A collection and transport device according to any preceding claim characterized in that the open end of the tube is provided with a collar (4) having an upper surface (3) which extends around the circumference of the tube and in that the collar (4) is designed for contact with the lower surface (6) of the closing means and with an intermediate sealing means (8).

5. A collection and transport device according to claim 4 characterized in that the lower circumference (6) of the closing means slopes inward and co-operates with an O-ring seal (8) placed against the contact surface (3) of the collar (4) which has a similar slope, thereby forming sharp contact edges which are pressed against the seal (8).

6. A collection and transport device according to any preceding claim characterized in that the plastic tube has an $O_2$ permeability coefficient < 30.

## Patentansprüche

1. Sammel- und Transportvorrichtung für Kulturen von Mikroorganismen mit einem Gefäß aus "unzerbrechlichem" transparenten Kunststoff, das einen Teils eines Mustertupfers des Typs enthält, der einen abbrechbaren Handgriff und einen Tupfer aus absorbierenden Fasern zum Aufnehmen einer Mikroorganismus-Kultur aufweist, und einem Verschlußmittel für das Gefäß, welches Mittel zum lösbaren Halten des Tupfers umfaßt,
**dadurch gekennzeichnet,**
daß das Gefäß eine einseitig offene Röhre (1) aus diffusionsfestem Werkstoff aufweist mit einem Permeabilitätskoeffizienten für $O_2 < 300$ und einer mechanischen Schlagfestigkeit derart, daß sich keine Risse in dem Werkstoff bilden, wenn er einer Schlagkraft von 60 kj bei 20°C ausgesetzt ist, wobei das Verschlußmittel (2) ebenfalls aus "unzerbrechlichem" und diffusionsfestem Werkstoff hergestellt ist, und daß die Länge der Röhre (1) geringer ist als die Länge des ungebrochenen Mustertupfers, wobei der Tupfer auf eine Länge abgebrochen wurde, die etwa der Länge der Röhre (1) entspricht, wobei ein Kulturenmedium in der Röhre (1) vorgesehen ist.

2. Sammel- und Transportvorrichtung nach Anspruch 1, dadurch gekennzeichnet, daß der Kunststoff Polyamid oder Polyethylen Terephtalat ist.

3. Sammel- und Transportvorrichtung nach einem der vorhergehenden Ansprüche, dadurch gekennzeichnet, daß das offene Ende der Röhre (1) mit einem Außengewinde versehen ist und daß entsprechende Verschlußmittel mit einem Innengewinde, und daß das Verschlußmittel mit einem mittigen, hohlen Haltestück (5) versehen ist, welches das obere Ende des Stiels eines Tupfers aufnimmt, wodurch der Tupfer in das Kulturenmedium hineingedreht wird, wenn das Verschlußmittel auf der Röhre (1) befestigt wird.

4. Sammel- und Transportvorrichtung nach einem der vorhergehenden Ansprüche, dadurch gekennzeichnet, daß das offene Ende der Röhre mit einem Kragen (4) versehen ist, der eine obere Fläche (3) aufweist, die sich um den Umfang der Röhre erstreckt, und daß der Kragen (4) die untere Fläche (6) des Verschlußmittels und ein Zwischendichtmittel (8) berührt.

5. Sammel- und Transportvorrichtung nach Anspruch 4, dadurch gekennzeichnet, daß der untere Umfang (6) des Verschlußmittels nach innen abgeschrägt ist und mit einem O-Ring (8) zusammenwirkt, der gegen die Berührungsfläche (3) des Kragens (4) angeordnet ist, welcher eine ähnliche Schrägung aufweist, wodurch scharfe Berührungskanten gebildet werden, die gegen die Dichtung (8) gepreßt sind.

6. Sammel- und Transportvorrichtung nach einem der vorhergehenden Ansprüche, dadurch gekennzeichnet, daß die Kunststoffröhre einen $O_2$ Permeabilitätskoeffizienten < 30 aufweist.

**Revendications**

1. Un procédé pour le transport et la collecte de cultures de micro-organismes comprenant un récipient en matériau plastique transparent et "incassable" contenant une partie d'un tampon-échantillon du type de celui comprenant un embout cassable et un tampon en fibres absorbantes pour collecter une culture de micro-organismes, et des moyens de fermeture du récipient comprenant des moyens de rétention non verrouillés du tampon caractérisés en ce que le récipient est constitué d'un tube ouvert à son extrémité (1) fait en matériau résistant à la diffusion avec un coefficient de perméabilité du $O_2$ < 300 et avec une force mécanique d'impact telle qu'aucune fêlure ne se forme lorsque ce matériau est soumis à une force d'impact de 60KJ à 20°C, le moyen de fermeture (2) étant aussi constitué d'un matériau incassable et résistant à la diffusion et en ce que la longueur du tube (1) est inférieure à celle du tampon-échantillon intact, le tampon ayant été détruit sur une longueur approximativement égale à celle du tube, et le bouillon de culture étant fourni avec le tube (1).

2. Un procédé pour la collecte et le transport selon la revendication 1, caractérisé en ce que le matériau plastique est du polyamide ou du polyéthylène téréphtalate.

3. Un procédé pour la collecte et le transport selon n'importe laquelle des revendications précédentes caractérisé en ce que l'ouverture du tube (1) est munie d'un pas de vis externe et les moyens de fermeture correspondants d'un pas de vis interne et en ce que les moyens de fermeture sont munis d'un réceptacle creux (5) conçu pour supporter l'extrémité supérieure de la tige du tampon qui permet la rotation de ce dernier dans le bouillon de culture lorsque le moyen de fermeture est serré sur le tube.

4. Un procédé pour la collecte et le transport selon n'importe laquelle des revendications précédentes caractérisé en ce que l'ouverture du tube est munie d'un collier (4) possédant une partie haute s'étendant à la circonférence du tube et en ce que le collier (4) est destiné à entrer en contact avec la partie basse (6) du moyen de fermeture et avec un moyen de scellement intermédiaire (8).

5. Un procédé pour la collecte et le transport selon la revendication 4, caractérisé en ce que la circonférence basse (6) du moyen de fermeture s' incline vers l'intérieur et coopère avec un anneau de serrage en "O" (8) placé contre la surface de contact (3) du collier (4) pourvu d'une inclinaison similaire, pour former des bords de contact très fins qui sont pressés contre le moyen de scellement (8).

6. Un procédé pour la collecte et le transport selon n'importe laquelle des revendications précédentes caractérisé en ce que le tube en plastique a un coefficient de perméabilité $O_2 <$ 30.

# Fig.1

# Fig.1a

# Fig. 2

a.                    b.                    c.